# EUROPEAN PATENT APPLICATION

(11) **EP 3 427 733 A1**
(43) Date of publication of application: **16.01.2019**
(21) Application number: 17763015.9
(22) Date of filing: 28.02.2017
(51) Int. Cl.: A61K 31/436, A61P 3/00, A61P 25/04, A61P 29/00

(54) **AGENT FOR TREATING FABRY DISEASE, ANALGESIC FOR EXTERNAL USE AND PERSPIRATION ACCELERATOR**

(30) Priority: 11.03.2016 JP 2016048356
(71) Applicant: Osaka University, Suita-shi, Osaka 565-0871 (JP)
(72) Inventor: KANEDA, Mari, Suita-shi Osaka 565-0871 (JP); KATAYAMA, Ichiro, Suita-shi Osaka 565-0871 (JP)
(74) Representative: Bohmann, Armin K.
(86) International application number: PCT/JP2017/007860
(87) International publication number: WO 2017/154675

(57) **Abstract**

An agent for treating Fabry disease, external analgesic, and perspiration enhancer in accordance with one embodiment of the present invention each contain rapamycin or a rapamycin derivative.

## Description

### Technical Field

The present invention relates to an agent for treating Fabry disease, an external analgesic, and a perspiration enhancer.

### Background Art

Fabry disease is a genetic disease caused by congenital deficiency of the lysosomal enzyme α-galactosidase (a-Gal). Due to the congenital deficiency of α-galactosidase (a-Gal), its substrates, i.e., glycolipids having galactose at their nonreducing end such as globotriaosylceramide (GL-3), galabiosylceramide (GL-2), and blood type B glycolipid, accumulate within lysosomes and, in turn, accumulate in various cells represented by vascular endothelial cells, cardiac muscle cells, and ganglion cells, resulting in development of various clinical symptoms related to all organs.

Depending on the organ affected by the accumulation of GL-3 and the like, various symptoms may appear such as cardiac dysfunction, renal dysfunction, nervous symptom including pain, angiokeratoma, and corneal clouding.

Accordingly, the symptoms of Fabry disease, which appear to be caused by anomalies of skin and its appendages such as sweat glands, also develop. One example is an anomaly of sweating. Especially in a case of a child patient, diminished sweating tends to cause heat accumulation or febrile convulsion, which may develop into severe conditions such as encephalopathy.

Usual analgesics are not so effective in relieving pains accompanying Fabry disease.

Angiokeratoma is one of the typical skin lesions of Fabry disease. Angiokeratoma frequently develops in any part of the body and, if it grows worse, even a slight stimulus will cause bleeding and the bleeding cannot be stopped easily in many cases. In addition, the bleeding area is a potential route of bacterial infection.

In treating Fabry disease, enzyme replacement therapy (ERT) has been conventionally used, by which a missing enzyme is externally supplied (Non-patent Literature 1).

Patent Literature 1 reports the effect of a TFEB phosphorylation inhibitor on lysosomal storage disease. Patent Literature 1 examines the effects of various compounds including rapamycin on lysosome or lysosome-related molecules with the use of typical cultured cells; however, Patent Literature 1 states that rapamycin is less effective in removing accumulated substances than other compounds such as Torin 1.

Patent Literature 2 discloses care of tuberous sclerosis complex using an external medicine containing sirolimus (rapamycin). Patent Literature 3 states that rapamycin suppresses perspiration.

Furthermore, Non-patent Literature 2 states that, as a result of oral administration of rapamycin (sirolimus) to a patient with neurofibroma, it was found that the pain-relieving effect is unclear and further studies are required.

### Citation List

### [Patent Literatures]

[Patent Literature 1] Japanese Translation of PCT Patent Application, Tokuhyo, No. 2014-511391 A (Publication Date: May 15, 2014)
[Patent Literature 2] Pamphlet of International Publication No. WO 2012/105521 (Publication Date: August 9, 2012)
[Patent Literature 3] Pamphlet of International Publication No. WO 2015/199248 (Publication Date: December 30, 2015)

### [Non-patent Literatures]

[Non-patent Literature 1] Genetics and Molecular Biology, 35, 4 (suppl), 947-954 (2012)
[Non-patent Literature 2] Neuro Oncol. 2015 Apr;17(4):596-603

### Summary of Invention

### Technical Problem

The ERT, which has conventionally been used to treat Fabry disease, is not so effective in treating the symptoms developed by a Fabry disease patient, especially skin lesions.

One aspect of the present invention was made in view of the foregoing issues of conventional techniques, and an object thereof is to provide an agent for treating Fabry disease that is effective in treating symptoms of Fabry disease, including skin lesions and nervous symptoms resistant to conventional treatments.

An object of another aspect of the present invention is to provide an external analgesic that is effective in treating pain.

An object of a further aspect of the present invention is to provide a treatment agent that is effective in treatment of diminished sweating (an agent for increasing perspiration).

### Solution to Problem

The inventors of the present invention studied hard in order to attain the above objects, and found that rapamycin and rapamycin derivatives are effective as agents for treating Fabry disease, that rapamycin-containing external medicines and rapamycin-derivative-containing external medicines are effective as analgesics, and that rapamycin and rapamycin derivatives are effective as perspiration enhancers. On the basis of these findings, the inventors accomplished the present invention.
<1> An agent for treating Fabry disease, the agent containing, as an active ingredient, at least one selected from the group consisting of rapamycin and rapamycin derivatives.
<2> The agent according to <1>, wherein the agent contains, as an active ingredient, at least one selected from the group consisting of rapamycin, everolimus, temsirolimus, ridaforolimus, and zotarolimus.
<3> The agent according to <1> or <2>, wherein the agent is an external medicine.
<4> The agent according to any one of <1> to <3>, wherein the agent is for use in treating a skin lesion of Fabry disease.
<5> An external analgesic containing, as an active ingredient, at least one selected from the group consisting of rapamycin and rapamycin derivatives.
<6> The external analgesic according to <5>, wherein the external analgesic contains, as an active ingredient, at least one selected from the group consisting of rapamycin, everolimus, temsirolimus, ridaforolimus, and zotarolimus.
<7> A perspiration enhancer containing, as an active ingredient, at least one selected from the group consisting of rapamycin and rapamycin derivatives.
<8> The perspiration enhancer according to <7>, wherein the perspiration enhancer contains, as an active ingredient, at least one selected from the group consisting of rapamycin, everolimus, temsirolimus, ridaforolimus, and zotarolimus.
<9> The perspiration enhancer according to <7> or <8>, wherein the perspiration enhancer is for treatment of anhidrosis or hypohidrosis.

### Advantageous Effects of Invention

Aspects of the present invention bring about an effect of treating various symptoms of Fabry disease, an effect of alleviating or controlling pain, and an effect of enhancing perspiration in hypohidrosis or anhidrosis.

An agent for treating Fabry disease in accordance with one embodiment of the present invention is capable of treating lesions that occur in any organ or tissue of a patient's body. The agent for treating Fabry disease is especially effective as a treatment agent for the treatment of symptoms of Fabry disease including skin lesions (e.g., pain in extremity, hypohidrosis, angiokeratoma). The use of the agent for treating Fabry disease in accordance with one embodiment of the present invention as an external medicine is effective especially in treating localized lesions (e.g., skin lesions).

An agent for treating Fabry disease, an external analgesic, and a perspiration enhancer, in accordance with one embodiment of the present invention, can be directly applied to a lesion. This eliminates the need for frequent visits to a hospital unlike ERT, enables easy application, and achieves retention of an active ingredient at high concentration at the lesion.

An agent for treating Fabry disease and an external analgesic in accordance with one embodiment of the present invention can each be in the form of topical preparation. Therefore, even if an active ingredient is present at a lesion at high concentration, the concentration of the active ingredient in blood is maintained relatively low, causing fewer adverse effects (e.g., side effects) and allowing for safe treatment.

### Brief Description of Drawings

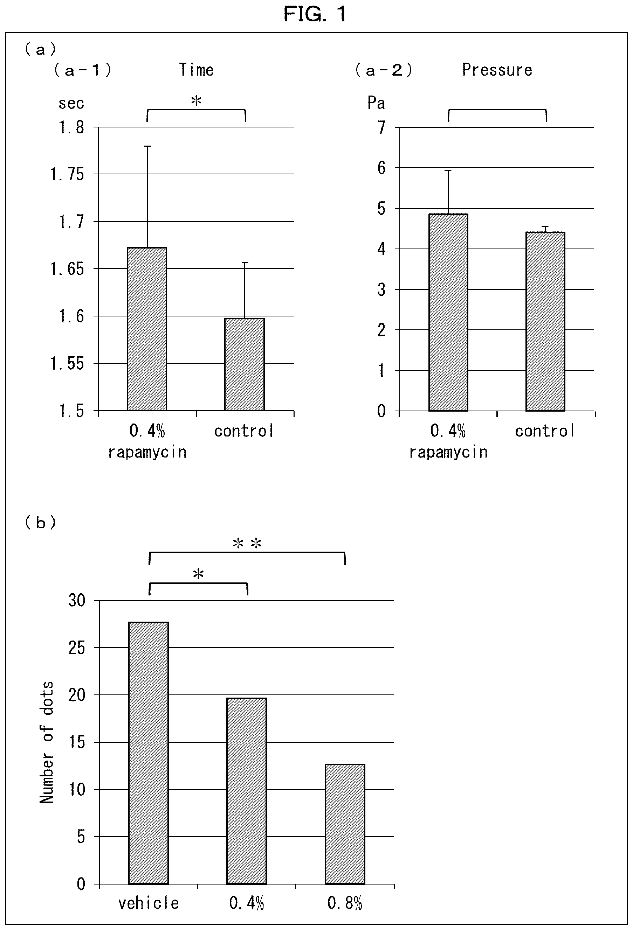

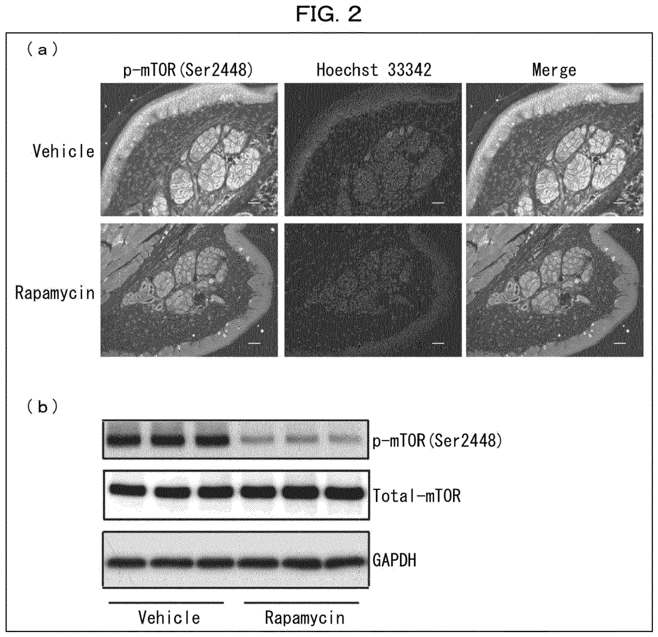

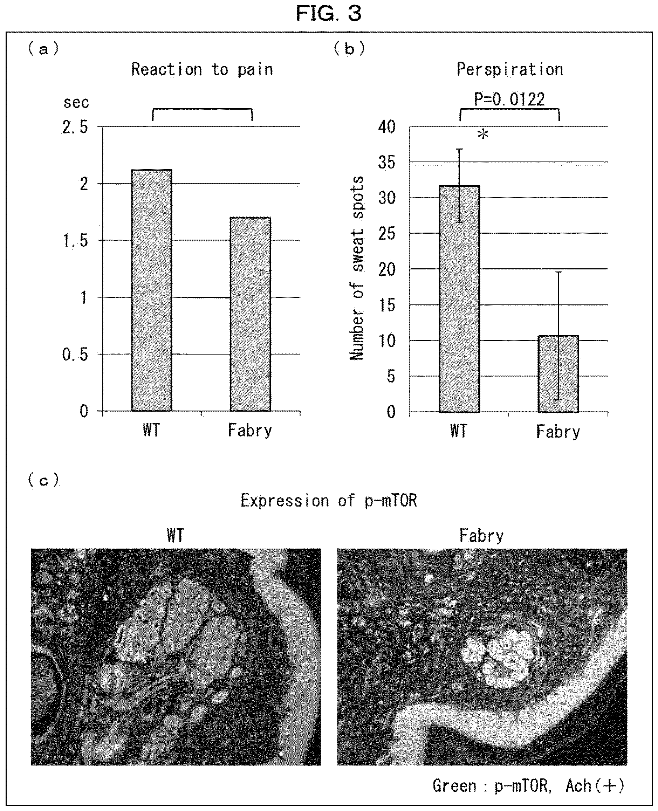

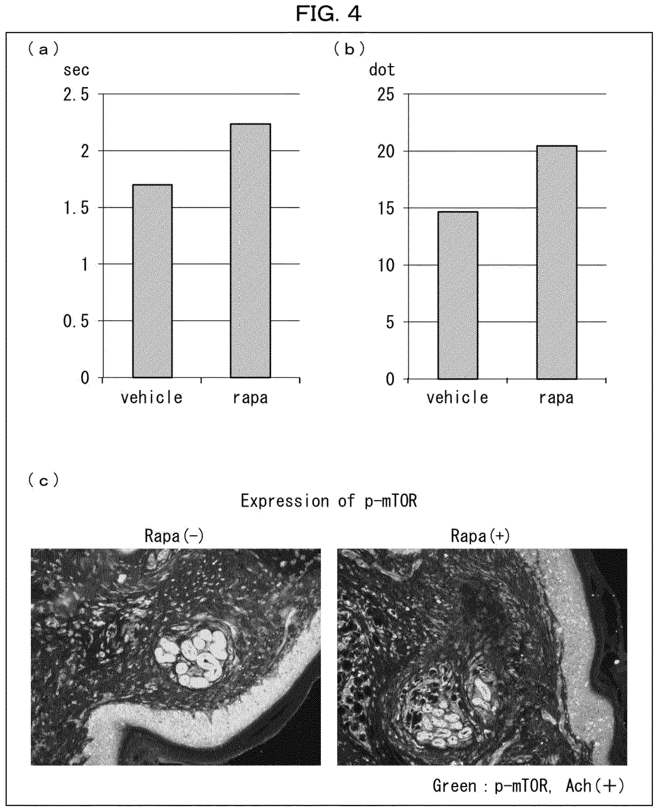

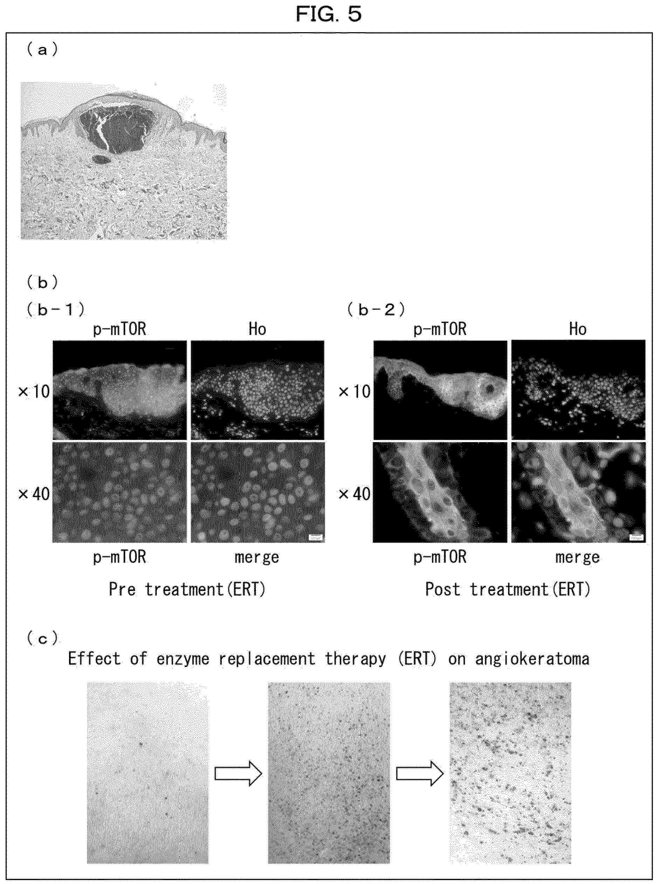

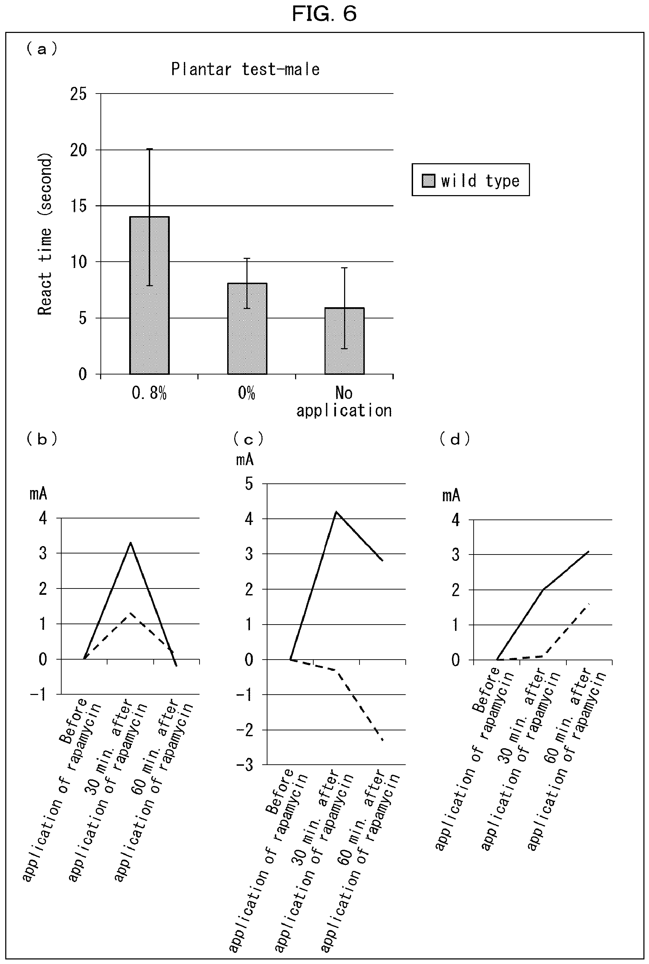

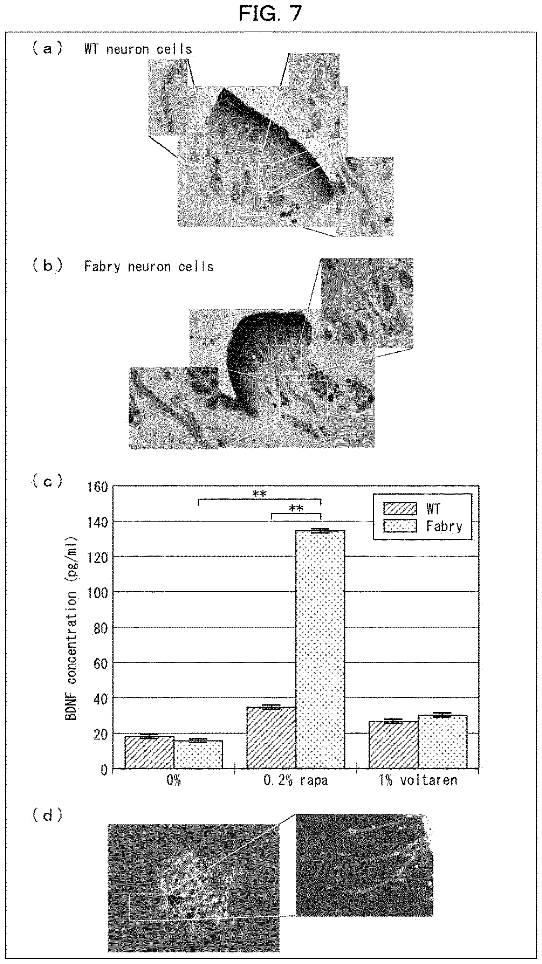

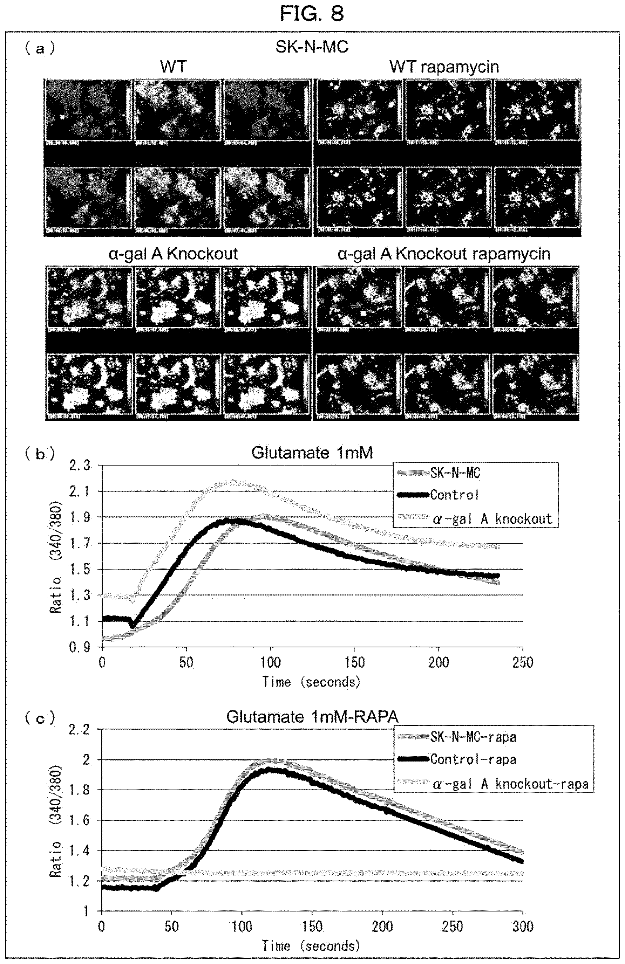

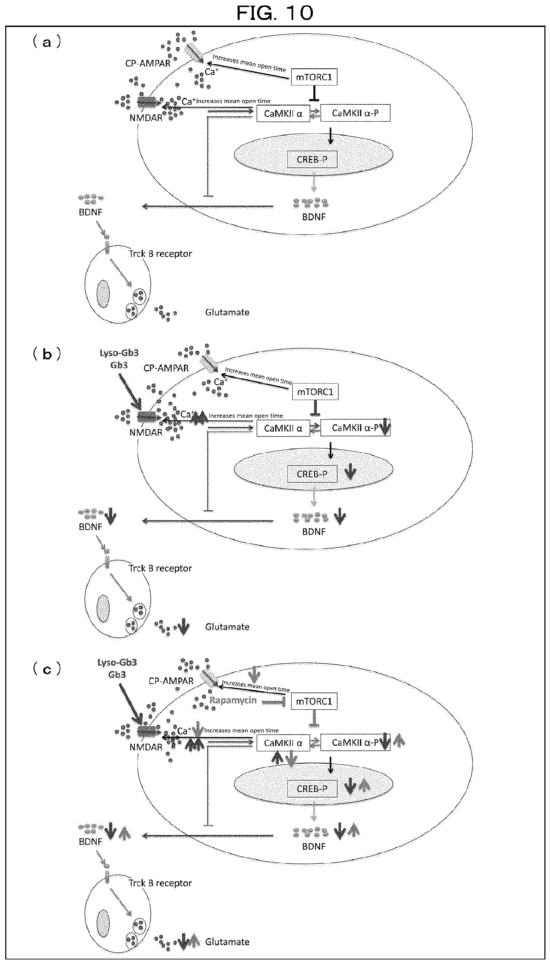

(a) of Fig. 1 shows graphs illustrating analgesic effect of rapamycin on wild-type mice. The graph (a-1) shows analgesic effect against thermal stimulation, and the graph (a-2) shows analgesic effect against pressure stimulation. (b) of Fig. 1 is a graph showing the antiperspirant effect of rapamycin on wild-type mice.
(a) of Fig. 2 shows fluorescent-stained images showing rapamycin's inhibitory effect on the expression of active mTOR (p-mTOR) in sweat glands of a wild-type mouse. (b) of Fig. 2 shows the results of western blotting showing rapamycin's inhibitory effect on the expression of active mTOR (p-mTOR) in sweat glands of a wild-type mouse.
(a) of Fig. 3 is a bar graph indicating that the pain threshold of Fabry disease model mice are lower than that of wild-type mice (the time that elapses before Fabry disease model mice avoid pain is shorter than that of wild-type mice). (b) of Fig. 3 is a bar graph indicating the symptom of diminished sweating in Fabry disease model mice. (c) of Fig. 3 shows fluorescent-stained images indicating that the expression of active mTOR (p-mTOR) is accelerated in sweat glands of a Fabry disease model mouse.
(a) of Fig. 4 is a bar graph indicating that the administration of rapamycin to Fabry disease model mice resulted in an increase in pain threshold (the time that elapsed before mice avoided pain was prolonged). (b) of Fig. 4 is a bar graph indicating that the administration of rapamycin to Fabry disease model mice resulted in an increase in perspiration. (c) of Fig. 4 shows fluorescent-stained images indicating that the administration of rapamycin resulted in inhibition of the expression of p-mTOR in sweat glands of a Fabry disease model mouse.
(a) of Fig. 5 is an image of a hematoxylin-eosin-stained angiokeratoma of a Fabry disease patient. (b-1) of Fig. 5 shows fluorescent-stained images indicating the expression of p-mTOR in an angiokeratoma lesion before receiving ERT. (b-2) of Fig. 5 shows fluorescent-stained images indicating the expression of p-mTOR in an angiokeratoma lesion after receiving ERT. The images indicate that p-mTOR was expressed both before and after ERT treatment. (c) of Fig. 5 show images indicating changes over time in angiokeratoma lesions that had received ERT over eight years.
(a) of Fig. 6 is a bar graph showing the analgesic effects in wild-type mice who received application of a rapamycin-containing external medicine . (b) to (d) of Fig. 6 are line graphs showing analgesic effects in healthy human subjects who received application of a rapamycin-containing external medicine. Solid lines indicate the results of application of 0.4% rapamycin, whereas dashed lines indicate the results of application of a base alone.
(a) of Fig. 7 is an image of stained neurons in a paw pad of a wild-type mouse. (b) of Fig. 7 is an image of stained neurons in a paw pad of a Fabry disease model mouse. (c) of Fig. 7 is a graph showing the brain-derived neurotrophic factor (BDNF) concentration in blood after (after 24 hours from) the application of rapamycin to wild-type mice and Fabry disease model mice. The graph indicates that the administration of rapamycin to Fabry disease model mice resulted in an increase in the BDNF concentration in blood of the Fabry disease model mice. (d) of Fig. 7 is a microscope image indicating that neurons in a dorsal root ganglion of a wild-type mouse grew in length due to BDNF.
(a) of Fig. 8 shows images showing the influx of calcium ions into human neuroblastoma cells (SK-N-MC). The results shown in (a) of Fig. 8 are: wild-type cells (upper left); rapamycin-administered wild-type cells (upper right); α-galA-knockout cells (lower left); and rapamycin-administered α-galA-knockout cells (lower right). (b) of Fig. 8 is a graph showing changes over time in influx of calcium ions into untreated cells, α-galA-knockout cells, and control cells. (c) of Fig. 8 is a graph showing changes over time in influx of calcium ions into rapamycin-administered untreated cells, rapamycin-administered α-galA-knockout cells, and rapamycin-administered control cells.

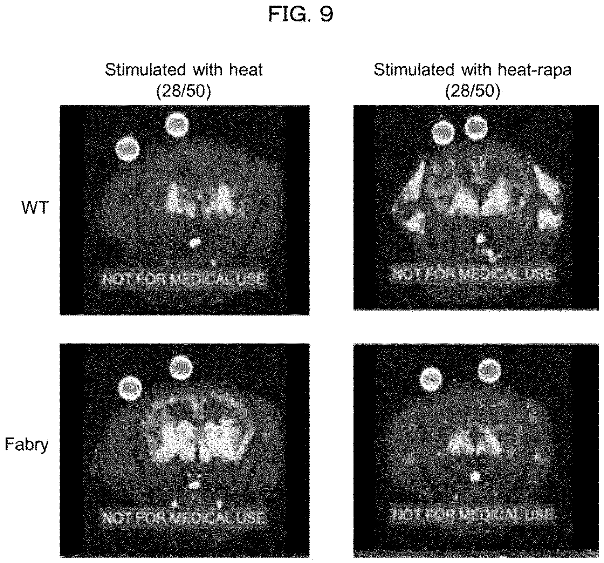

Fig. 9 shows MRI images showing the activity of the brains of a wild-type mouse and a Fabry disease model mouse who received thermal stimulation. The results shown in Fig. 9 are: the results for a mouse without treatment (left); and the results for a rapamycin-administered mouse (right).
(a) of Fig. 10 is a model diagram showing a nerve cell in central nervous system in normal state. (b) of Fig. 10 is a model diagram showing a presumed action mechanism of how influx of excessive calcium ions into a nerve cell in central nervous system occurs in a Fabry disease patient. (c) of Fig. 10 is a model diagram showing a presumed action mechanism of rapamycin (sirolimus) in one aspect of the present invention.

### Description of Embodiments

The present invention is not limited to the following embodiments, but can be altered by a skilled person in the art within the scope of the claims. The present invention also encompasses, in its technical scope, any embodiment or example derived by combining technical means disclosed in differing embodiments or examples.

The expression "A to B" representing a numeric range herein refers to "not less than A and not more than B", unless otherwise specifically noted.

An agent for treating Fabry disease, an external analgesic, and a perspiration enhancer, in accordance with one embodiment of the present invention, are medicaments for providing a Fabry disease treating effect, a pain-relieving effect, and a perspiration enhancing effect, respectively, and each contain, as an active ingredient, at least one selected from the group consisting of rapamycin and rapamycin derivatives.

Rapamycin and rapamycin derivative have been conventionally used also in treating other diseases, and their safety has been clinically established. Therefore, the agent for treating Fabry disease and the external analgesic of the present embodiment have a high level of safety.

The rapamycin derivatives are not limited to a particular kind. Examples of the rapamycin derivatives include everolimus, temsirolimus, ridaforolimus, and zotarolimus. These rapamycin derivatives are known to have substantially the same basic structure as that of rapamycin and thus have physiological activities equivalent to that of rapamycin. Therefore, these rapamycin derivatives can be used as the active ingredient(s) in accordance with one embodiment of the present invention, as with rapamycin.

Among the above-listed rapamycin and rapamycin derivatives, rapamycin, whose percutaneous absorption has been confirmed, is more preferable than everolimus and the like, which have a high molecular weight and are soluble in water and thus normally appear to be hardly absorbed percutaneously, from the viewpoint of preparation of an external medicine.

An agent for treating Fabry disease, an external analgesic, and a perspiration enhancer, in accordance with one embodiment of the present invention, can be used not only for humans but also for non-human mammals. Examples of non-human mammals include mammals excluding humans. Examples of mammals excluding humans include: cloven-hoofed animals such as cattle, boars, pigs, sheep, and goats; ungulates such as horses; rodents such as mice, rats, hamsters, and squirrels; lagomorphs such as rabbits; and carnivorous animals such as dogs, cats, and ferrets. These non-human animals are not limited to barn animals or companion animals (pet animals), and may be wild animals.

### [(I) Agent for treating Fabry disease]

An agent for treating Fabry disease in accordance with one embodiment of the present invention can be used to treat various symptoms that a Fabry disease patient may develop. Examples of the symptoms of Fabry disease include skin lesions, nervous symptoms, decreased renal function, decreased cardiac function, eye symptoms (such as corneal lesions), ear symptoms (such as auditory disorder), and digestive symptoms (such as diarrhea and abdominal pain).

The term "treatment" herein refers to any action that brings about a treatment effect. The concept of "treatment effect" includes a prophylactic effect and a curing effect. The treatment effect is, for example, at least one of the effects of the following types.
(1) Development of one or more symptoms of the disease is prevented or the risk of development of one or more symptoms of the disease is reduced, as compared to cases in which no treatment agents are administered.
(2) Relapse of one or more symptoms of the disease is prevented or the risk of relapse of one or more symptoms of the disease is reduced, as compared to cases in which no treatment agents are administered.
(3) Signs of one or more symptoms of the disease are prevented from occurring or the risk of occurrence of signs of one or more symptoms of the disease is reduced, as compared to cases in which no treatment agents are administered.
(4) Severity of one or more symptoms of the disease is reduced (one or more symptoms are alleviated) or one or more symptoms are caused to disappear, as compared to cases in which no treatment agents are administered.
(5) Severity of one or more symptoms of the disease is prevented from increasing or one or more symptoms are prevented from progressing (one or more symptoms are prevented from worsening), as compared to cases in which no treatment agents are administered.
(6) Rate of increase of severity or rate of progress of one or more symptoms of the disease is reduced (worsening of one or more symptoms is alleviated), as compared to cases in which no treatment agents are administered.

An agent for treating Fabry disease in accordance with one embodiment of the present invention can be suitably used in treating (e.g., curing or prevention of) skin lesions of Fabry disease. Therefore, the agent for treating Fabry disease in accordance with one embodiment of the present invention can be a curative agent for Fabry disease or a prophylactic agent for Fabry disease.

The term "skin lesion of Fabry disease" herein includes not only typical skin lesions but also other symptoms such as pains in skin and nearby tissues (e.g., pain in extremity, acroparesthesia), diminished sweating, absent sweating, and angiokeratoma.

An agent for treating Fabry disease in accordance with one embodiment of the present invention can be more suitably used in treating pains in skin and nearby tissues, diminished sweating, absent sweating, and angiokeratoma, than in treating other skin lesions of Fabry disease.

The inventors previously found that rapamycin has an antiperspirant effect (see Patent Literature 3). However, surprisingly, it was found that, when used for Fabry disease, rapamycin has a perspiration enhancing effect, instead of the antiperspirant effect.

The route of administration of the agent for treating Fabry disease in accordance with one embodiment of the present invention is not limited, and may be any route such as oral, percutaneous, enteral, intravenous, or transmucosal route. Therefore, the agent for treating Fabry disease in accordance with one embodiment of the present invention can be in the form of, for example, internal medicine, external medicine, injectable medicine, suppository, or inhalant. The agent for treating Fabry disease in accordance with one embodiment of the present invention is preferably in the form of external medicine or internal medicine, more preferably in the form of external medicine.

The agent in any form can be used to effectively treat Fabry disease. With the agent in the form of external medicine, a local lesion (e.g., skin lesion) of Fabry disease of a living body can be easily treated. On the other hand, with the agent in the form of internal medicine, symptoms of Fabry disease in any part of a living body (e.g., troubles of internal organs, skin lesion) can be easily treated. Topical administration of the external medicine makes it possible to retain the active ingredient at the lesion at high concentration and thereby achieve a desired effect, and does not cause significant side effects because the amount of the agent moving into blood is small. As such, external medicines are preferred from safety point of view.

The following description will first specifically discuss an agent for treating Fabry disease in the form of external medicine, and then specifically discuss an agent for treating Fabry disease in the form of internal medicine.

### (I-A) External medicine

The following description will discuss an agent for treating Fabry disease in the form of external medicine.

Examples of the external medicine include gel preparations, ointments, patches, poultices, liniments, lotions, and creams. Gel preparations are more preferred because their active ingredients are more easily absorbed into skin tissue as compared to ointments and the like.

The amount of an active ingredient contained in the external medicine in accordance with one embodiment of the present invention is not particularly limited. The following concentrations are preferred, in order to reduce systemic side effects and achieve a treatment effect by reducing the amount of the active ingredient moving into blood while increasing the concentration of the active ingredient in skin tissue to a therapeutically effective concentration.

The lower limit of the concentration of the active ingredient may be not less than 0.01 wt%, not less than 0.02 wt%, not less than 0.03 wt%, not less than 0.04 wt%, not less than 0.05 wt%, not less than 0.06 wt%, not less than 0.07 wt%, not less than 0.08 wt%, not less than 0.09 wt%, not less than 0.1 wt%, relative to the total weight of the external medicine.

The upper limit of the concentration of the active ingredient may be not more than 1.0 wt%, not more than 0.9 wt%, not more than 0.8 wt%, not more than 0.7 wt%, not more than 0.6 wt%, not more than 0.5 wt%, not more than 0.4 wt%, not more than 0.3 wt%, not more than 0.25 wt%, not more than 0.2 wt%, relative to the total weight of the external medicine.

The concentration of the active ingredient is, for example, preferably 0.01 wt% to 1.0 wt%, more preferably 0.02 wt% to 0.9 wt%, more preferably 0.03 wt% to 0.8 wt%, more preferably 0.04 wt% to 0.7 wt%, more preferably 0.05 wt% to 0.6 wt%, more preferably 0.06 wt% to 0.5 wt%, more preferably 0.07 wt% to 0.4 wt%, more preferably 0.08 wt% to 0.3 wt%, more preferably 0.09 wt% to 0.25 wt%, most preferably 0.1 wt% to 0.2 wt%, relative to the total weight of the external medicine. The lower limit of each of the above ranges of concentration may be changed to 0.05 wt%. The amount of rapamycin or a rapamycin derivative may be 0.4 wt% to 0.8 wt%.

The upper limit of the amount of rapamycin or a rapamycin derivative contained in the external medicine is not particularly limited, and is, for example, preferably 0.8 wt%, more preferably 0.4 wt%.

The dosage of rapamycin or a rapamycin derivative per unit surface area of a living body per day is not particularly limited.

The lower limit of the dosage per day may be not less than 0.0001 mg/cm², not less than 0.0002 mg/cm², not less than 0.0005 mg/cm², not less than 0.001 mg/cm², not less than 0.002 mg/cm², not less than 0.003 mg/cm².

The upper limit of the dosage per day may be not more than 2 mg/cm², not more than 1 mg/cm², not more than 0.5 mg/cm², not more than 0.05 mg/cm², not more than 0.04 mg/cm², not more than 0.03 mg/cm².

The dosage is, for example, 0.0001 mg/cm² to 2 mg/cm², preferably 0.0002 mg/cm² to 1 mg/cm², more preferably 0.0005 mg/cm² to 0.5 mg/cm², more preferably 0.001 mg/cm² to 0.05 mg/cm², more preferably 0.002 mg/cm² to 0.04 mg/cm², even more preferably 0.003 mg/cm² to 0.03 mg/cm². Rapamycin or a rapamycin derivative in any of the above listed amounts may be administered once a day (by application etc.) or may be administered in several doses in a day. In other words, it is preferable that the external medicine in accordance with one embodiment of the present invention is in the form that can achieve any of the above described dosages.

Examples of a method of preparing an external medicine are as follows. (i) A gel preparation can be prepared by allowing a solution containing rapamycin or a rapamycin derivative to gelate. (ii) An ointment can be prepared by mixing an ointment base with rapamycin or a rapamycin derivative. (iii) A patch, poultice, liniment, lotion, and cream can be prepared in accordance with known methods.

The following description will specifically discuss the gel preparation and ointment.

### (I-A-1) Gel preparation

As described above, an agent for treating Fabry disease in accordance with one embodiment of the present invention may be a gel preparation that can be obtained by allowing a solution containing rapamycin or a rapamycin derivative to gelate.

The gelation of a solution containing rapamycin or a rapamycin derivative can be achieved by allowing the solution to gelate with the use of a gelation inducer. Examples of the gelation inducer include carboxyvinyl polymer, carboxymethyl cellulose, aluminum hydroxide, and bentonite.

Carboxyvinyl polymer used here is not particularly limited as to its specific structure, and can be Carbopol (registered trademark), HIVISWAKO (registered trademark), or AQUPEC (registered trademark). Among these kinds of carboxyvinyl polymer, Carbopol (registered trademark) 934P NF and Carbopol (registered trademark) 980 are preferred for good texture of the resulting external medicine.

For example, in a case where Carbopol (registered trademark) is used, Carbopol (registered trademark) is first added to a solution containing rapamycin or a rapamycin derivative. Then, a pH adjuster (e.g., tris(hydroxymethyl)aminomethane, triethanolamine) is added, and thereby the pH of the solution is adjusted to neutral. This can induce the gelation of the solution.

The gel preparation in accordance with one embodiment of the present invention preferably contains an alcohol. An alcohol can cause the active ingredient contained in the gel preparation to be efficiently absorbed into skin tissue and efficiently delivered to an affected site. Examples of the alcohol include ethanol and isopropanol. For achieving more efficient absorption of the active ingredient into skin tissue, ethanol is preferred.

The amount of the alcohol is not particularly limited, provided that the amount is sufficient to dissolve rapamycin or a rapamycin derivative. For example, the weight of the alcohol may be 100 times to 300 times the weight of the rapamycin or the rapamycin derivative, may be 100 times to 300 times the weight of the rapamycin or the rapamycin derivative, or may be 120 times to 250 times the weight of the rapamycin or the rapamycin derivative. Relative to the total weight of the external medicine, the amount of the alcohol is preferably not less than 20 wt%, more preferably not less than 30 wt%, even more preferably not less than 40 wt%. Provided that the amount of the alcohol is not more than 60 wt%, the alcohol in the resulting preparation will not be lost greatly from evaporation, and the preparation with stable active ingredient concentration can be preserved (stored). As such, the amount of the alcohol is more preferably about 50 wt% (45% to 55%).

The alcohol contained in an amount that is sufficient to dissolve the active ingredient allows the active ingredient to be more efficiently absorbed into skin tissue. Provided that the amount of the alcohol is not more than 50 wt%, a larger amount of the alcohol better dissolves the active ingredient, and therefore the active ingredient is more efficiently absorbed into skin tissue.

The amount of the gelation inducer contained in the gel preparation is not particularly limited, provided that the amount is sufficient to allow the solution containing rapamycin or a rapamycin derivative to gelate.

The amount of the gelation inducer contained in the gel preparation is not particularly limited, provided that the amount is sufficient to allow the solution containing rapamycin or a rapamycin derivative to gelate. The amount of the gelation inducer contained in the gel preparation may be, for example, not less than 1.5 wt% relative to the total weight of the gel preparation. More specifically, the amount of the gelation inducer contained in the gel preparation may be 1.5 wt% to 20 wt%, 1.5 wt% to 15 wt%, 1.5 wt% to 10 wt%, 1.5 wt% to 5 wt%, or 1.5 wt% to 2.5 wt%, relative to the total weight of the gel preparation.

The amount of the pH adjuster (neutralizer) contained in the gel preparation is not particularly limited, and may be selected depending on the amounts of a solvent and the gelation inducer. The amount of the pH adjuster contained in the gel preparation may be, for example, 0.5 wt% to 5.0 wt%, 0.5 wt% to 2.5 wt%, or 0.5 wt% to 1.0 wt%, relative to the total weight of the gel preparation.

For example, in a case where components that are used to induce gelation are a gelation inducer (such as Carbopol (registered trademark)) and a pH adjuster (such as tris(hydroxymethyl)aminomethane), the amount of the gelation inducer may be, for example, 1.6 wt% relative to the total weight of the external medicine, and the amount of the pH adjuster may be, for example, 0.4 wt%, 0.6 wt% or 0.8 wt% relative to the total weight of the external medicine. It should be understood that the present invention is not limited to such percentages.

The gel preparation may contain some other component(s) in addition to the foregoing active ingredient, solvent (alcohol), gelation inducer, and pH adjuster (neutralizer). Examples of such other component(s) include water-soluble polymers, water, and medicinal components other than rapamycin and rapamycin derivatives.

Examples of the water-soluble polymers include polyethylene glycol, starch, methylcellulose, hydroxypropylcellulose (HPC), polyvinyl alcohol, polyvinyl methyl ether, and polyvinyl pyrrolidone.

In a case where the gel preparation contains hydroxypropylcellulose, the viscosity of the gel preparation improves. That is, the gel preparation does not easily come off of the skin.

The amount of the foregoing other component(s) contained in the gel preparation is not particularly limited, and may be, for example, not more than 50 wt%, not more than 40 wt%, not more than 30 wt%, not more than 20 wt%, not more than 10 wt%, relative to the total weight of the gel preparation.

The amount of rapamycin or a rapamycin derivative contained in the gel preparation is not particularly limited; however, in order to prevent systemic side effects by reducing the amount of the active ingredient moving into blood and concurrently to obtain a treatment effect by increasing the concentration of the active ingredient in skin tissue to a therapeutically effective concentration, the following concentrations are preferred.

The lower limit of the amount of rapamycin or a rapamycin derivative may be not less than 0.01 wt%, not less than 0.02 wt%, not less than 0.03 wt%, not less than 0.04 wt%, not less than 0.05 wt%, not less than 0.06 wt%, not less than 0.07 wt%, not less than 0.08 wt%, not less than 0.09 wt%, not less than 0.1 wt%, relative to the total weight of the gel preparation.

The upper limit of the amount of rapamycin or a rapamycin derivative may be not more than 1.0 wt%, not more than 0.9 wt%, not more than 0.8 wt%, not more than 0.7 wt%, not more than 0.6 wt%, not more than 0.5 wt%, not more than 0.4 wt%, not more than 0.3 wt%, not more than 0.25 wt%, not more than 0.2 wt%, relative to the total weight of the gel preparation.

The amount of rapamycin or a rapamycin derivative is, for example, preferably 0.01 wt% to 1.0 wt%, more preferably 0.02 wt% to 0.9 wt%, more preferably 0.03 wt% to 0.8 wt%, more preferably 0.04 wt% to 0.7 wt%, more preferably 0.05 wt% to 0.6 wt%, more preferably 0.06 wt% to 0.5 wt%, more preferably 0.07 wt% to 0.4 wt%, more preferably 0.08 wt% to 0.3 wt%, more preferably 0.09 wt% to 0.25 wt%, most preferably 0.1 wt% to 0.2 wt%, relative to the total weight of the gel preparation. The lower limit of each of the above ranges of concentration may be changed to 0.05 wt%. The amount of rapamycin or a rapamycin derivative may be 0.4 wt% to 0.8 wt%.

The upper limit of the amount of rapamycin or a rapamycin derivative contained in the gel preparation is not particularly limited, and is, for example, preferably 0.8 wt%, more preferably 0.4 wt%.

With rapamycin or a rapamycin derivative contained in any of the above-listed amounts, the foregoing symptoms of Fabry disease can be effectively treated, and also the resultant agent for treating Fabry disease has a high level of safety.

The lower limit of the amount of the gel preparation applied per unit surface area of a living body may be not less than 0.001 g/cm², not less than 0.002 g/cm², not less than 0.003 g/cm², not less than 0.004 g/cm², not less than 0.005 g/cm².

The upper limit of the amount of the gel preparation applied per unit surface area of a living body may be not more than 0.01 g/cm², not more than 0.009 g/cm², not more than 0.008 g/cm², not more than 0.007 g/cm², not more than 0.006 g/cm².

The amount of the gel preparation applied per unit surface area of a living body is not particularly limited, and may be 0.001 g/cm² to 0.01 g/cm², 0.002 g/cm² to 0.009 g/cm², 0.003 g/cm² to 0.008 g/cm², 0.004 g/cm² to 0.007 g/cm², 0.005 g/cm² to 0.006 g/cm².

The gel preparation may be applied in any of the above amounts every day or once in two or three days. It is preferable that the gel preparation is applied every day. In a case where the gel preparation is applied every day, the gel preparation is applied preferably once to three times a day, more preferably twice to three times a day, and most preferably twice a day.

It is preferable that the concentrations of components in the preparation and the frequency of administration of the preparation are adjusted suitably depending on the age, administration site, thickness of the skin, and the like. For example, to a young child's thin skin, armpit, or the like, the gel preparation having an active ingredient concentration of 0.05% to 0.2% can be applied once to three times a day. To a palm, a sole, or the like, the gel preparation having an active ingredient concentration of 0.2% to 0.8% can be applied once to twice a day.

The following description will discuss more specific examples of the composition of the gel preparation. Note, however, that the present invention is not limited to such compositions. With the arrangements described below, it is possible to further enhance the Fabry disease treatment effect brought about by rapamycin or a rapamycin derivative. Furthermore, with the arrangements described below, the amount of rapamycin or a rapamycin derivative required to achieve a desired effect is smaller. This reduces adverse effects to living bodies.

The gel preparation may contain Carbopol (registered trademark) 934P NF, water, an alcohol (e.g., ethanol or isopropanol, preferably, ethanol), and tris(hydroxymethyl)aminomethane, in addition to rapamycin or a rapamycin derivative.

In a case where the gel preparation contains Carbopol (registered trademark) 934P NF, water, an alcohol, and tris(hydroxymethyl)aminomethane, the weight ratio among rapamycin or a rapamycin derivative, Carbopol (registered trademark) 934P NF, water, alcohol, and tris(hydroxymethyl)aminomethane may be (0.5 to 2) : 16 : 490 : (450 to 500) : 6.

The above ratio may alternatively be as follows. Rapamycin or a rapamycin derivative : Carbopol (registered trademark) 934P NF : water : alcohol : tris(hydroxymethyl)aminomethane = (0.5 to 2) : 16 : 490 : (480 to 490) : 6. The ratio may alternatively be as follows. Rapamycin or a rapamycin derivative : Carbopol (registered trademark) 934P NF : water : alcohol : tris(hydroxymethyl)aminomethane = 2 : 16 : 490 : 486 : 6.

### (I-A-2) Ointment

As described earlier, an agent for treating Fabry disease in accordance with one embodiment of the present invention may be an ointment obtained by adding rapamycin or a rapamycin derivative to an ointment base.

Examples of a base include waxes (e.g., natural waxes such as white beeswax, lanolin, carnauba wax, and spermaceti wax; mineral waxes such as montan wax; and synthetic waxes); paraffins (e.g., liquid paraffin, solid paraffin, and the like); petrolatums (e.g., white petrolatum, yellow petrolatum, and the like); and the like.

The amount of the base is not particularly limited, and may be, for example, not less than 10 wt%, not less than 20 wt%, not less than 30 wt%, not less than 40 wt%, not less than 50 wt%, not less than 60 wt%, not less than 70 wt%, not less than 80 wt%, not less than 90 wt%, relative to the total weight of the ointment.

The ointment may contain some other component(s) in addition to rapamycin or a rapamycin derivative. Examples of such other component(s) include the components described in the foregoing section (I-A-1).

The ointment may contain propylene carbonate, solid paraffin, and white petrolatum, in addition to rapamycin or a rapamycin derivative. The ointment may also contain liquid paraffin, in addition to propylene carbonate, solid paraffin, and white petrolatum. The ointment may also contain white beeswax, in addition to propylene carbonate, solid paraffin, white petrolatum, and liquid paraffin.

In a case where the ointment contains propylene carbonate, solid paraffin, white petrolatum, liquid paraffin, and white beeswax in addition to rapamycin or a rapamycin derivative, the weight ratio among rapamycin or a rapamycin derivative, propylene carbonate, solid paraffin, white petrolatum, liquid paraffin, and white beeswax may be (0.3 to 10) : (50 to 59.4) : (30 to 45) : 895 : (0 to 10) : (0 to 5), with the proviso that the active ingredient, propylene carbonate, solid paraffin, and liquid paraffin are 105 in total.

More specifically, the above ratio may be Ratio 1, Ratio 2, or Ratio 3 shown below.
(Ratio 1) Rapamycin or rapamycin derivative : propylene carbonate : solid paraffin : white petrolatum : liquid paraffin : white beeswax = 2 : 58 : 30 : 895 : 10 : 5
(Ratio 2) Rapamycin or rapamycin derivative : propylene carbonate : solid paraffin : white petrolatum : liquid paraffin : white beeswax = 2 : 58 : 45 : 895 : 0 : 0
(Ratio 3) Rapamycin or rapamycin derivative : propylene carbonate : solid paraffin : white petrolatum : liquid paraffin : white beeswax = 2 : 58 : 35 : 895 : 10 : 0

When a comparison is made between Ratio 1 and Ratio 2 above, an external medicine prepared in accordance with Ratio 1 is more transparent and has less water in its surface than an external medicine prepared in accordance with Ratio 2.

When a comparison is made between Ratio 1 and Ratio 3 above, the external medicine prepared in accordance with Ratio 1 is smoother and has less water in its surface than an external medicine prepared in accordance with Ratio 3.

The amount of rapamycin or a rapamycin derivative contained in the ointment is not particularly limited; however, in order to prevent systemic side effects by reducing the amount of the active ingredient moving into blood and to concurrently obtain a treatment effect by increasing the concentration of the active ingredient in skin tissue to a therapeutically effective concentration, the following concentrations are preferred.

The lower limit of the amount of rapamycin or a rapamycin derivative may be not less than 0.01 wt%, not less than 0.02 wt%, not less than 0.03 wt%, not less than 0.04 wt%, not less than 0.05 wt%, not less than 0.06 wt%, not less than 0.07 wt%, not less than 0.08 wt%, not less than 0.09 wt%, not less than 0.1 wt%, relative to the total weight of the ointment.

The upper limit of the amount of rapamycin or a rapamycin derivative may be not more than 1.0 wt%, not more than 0.9 wt%, not more than 0.8 wt%, not more than 0.7 wt%, not more than 0.6 wt%, not more than 0.5 wt%, not more than 0.4 wt%, not more than 0.3 wt%, not more than 0.25 wt%, not more than 0.2 wt%, relative to the total weight of the ointment.

The amount of rapamycin or a rapamycin derivative is, for example, preferably 0.01 wt% to 1.0 wt%, more preferably 0.02 wt% to 0.9 wt%, more preferably 0.03 wt% to 0.8 wt%, more preferably 0.04 wt% to 0.7 wt%, more preferably 0.05 wt% to 0.6 wt%, more preferably 0.06 wt% to 0.5 wt%, more preferably 0.07 wt% to 0.4 wt%, more preferably 0.08 wt% to 0.3 wt%, more preferably 0.09 wt% to 0.25 wt%, most preferably 0.1 wt% to 0.2 wt%, relative to the total weight of the ointment. The lower limit of each of the above ranges of concentration may be changed to 0.05 wt%. The amount of rapamycin or a rapamycin derivative may be 0.4 wt% to 0.8 wt%.

The upper limit of the amount of rapamycin or a rapamycin derivative contained in the ointment is not particularly limited, and is, for example, preferably 0.8 wt%, more preferably 0.4 wt%.

With rapamycin or a rapamycin derivative contained in any of the above-listed amounts, the foregoing symptoms of Fabry disease can be effectively treated, and also the resultant agent for treating Fabry disease has a high level of safety.

The lower limit of the amount of the ointment applied per unit surface area of a living body may be not less than 0.001 g/cm², not less than 0.002 g/cm², not less than 0.003 g/cm², not less than 0.004 g/cm², not less than 0.005 g/cm².

The upper limit of the amount of the ointment applied per unit surface area of a living body may be not more than 0.01 g/cm², not more than 0.009 g/cm², not more than 0.008 g/cm², not more than 0.007 g/cm², not more than 0.006 g/cm².

The amount of the ointment applied per unit surface area of a living body is not particularly limited, and may be 0.001 g/cm² to 0.01 g/cm², 0.002 g/cm² to 0.009 g/cm², 0.003 g/cm² to 0.008 g/cm², 0.004 g/cm² to 0.007 g/cm², 0.005 g/cm² to 0.006 g/cm².

The ointment may be applied in any of the above amounts every day or once in two or three days. It is preferable that the ointment is applied every day. In a case where the ointment is applied every day, the ointment is applied preferably once to three times a day, more preferably twice to three times a day, and most preferably twice a day.

It is preferable that the concentrations of components in the preparation and the frequency of administration of the preparation are adjusted suitably depending on the age, administration site, thickness of the skin, and the like. For example, to a young child's thin skin, armpit, or the like, the ointment having an active ingredient concentration of 0.05% to 0.2% can be applied once to three times a day. To a palm, a sole, or the like, the ointment having an active ingredient concentration of 0.2% to 0.8% can be applied once to twice a day.

The ointment can be produced by a known method. The following description will discuss an example of the production method.

The ointment can be prepared by using, for example, a homomixer (for example, a homomixer manufactured by PRIMIX Corporation) or a universal mixer (for example, a universal mixer manufactured by Dalton Corporation). In a case where the base is solid at room temperature, the base may be heated until the base liquefies and the base in liquid state may be mixed with a solution in which the active ingredient is dissolved. More specifically, the ointment can be produced by: melting various components (e.g., waxes, paraffins, petrolatums, etc.) that are solid at room temperature by heating the components to a temperature (e.g., 70°C) higher than the melting points of the components; adding, to the components thus melted, a solution in which the active ingredient is dissolved and stirring the mixture thus obtained; and then cooling the mixture to substantially room temperature (e.g., 40°C) with stirring.

Another method of producing the ointment is as follows. The entire base is melted at a temperature of 70°C to 80°C, and, by use of a planetary centrifugal mixer (manufactured by Thinky Corporation), the base is stirred, in a stirring mode, at 800 rpm for 30 minutes, next at 1000 rpm for 5 minutes, and then at 2000 rpm for 1 minute (15°C). Then, a solution in which the active ingredient is dissolved is added to the base, and further stirred at 1000 rpm for 1 minute and then at 2000 rpm for 1 minute (without cooling). This makes it possible to prepare a better-quality ointment in which very fine particles containing the active ingredient are dispersed.

In a case where the ointment contains other component(s) described earlier, the ointment may be prepared by: preparing a solution by dissolving the active ingredient and the other components(s) in a certain solvent; adding a base to the solution; and then carrying out subsequent steps in accordance with the method described above.

### (I-B) Internal medicine

The following description will discuss an agent for treating Fabry disease in the form of internal medicine.

An agent for treating Fabry disease in the form of internal medicine can be prepared by mixing rapamycin or a rapamycin derivative with a component(s) for use in a known internal medicine.

The amount of rapamycin or a rapamycin derivative contained in one dose of the internal medicine is not particularly limited, and can be appropriately selected depending on the subject who receives the administration.

The lower limit of the dosage of rapamycin or a rapamycin derivative per adult per day may be not less than 0.1 mg, not less than 0.2 mg, not less than 0.4 mg, not less than 0.8 mg, not less than 1 mg, not less than not less than 2 mg.

The upper limit of the dosage of rapamycin or a rapamycin derivative per adult per day may be not more than 10 mg, not more than 9 mg, not more than 8 mg, not more than 7 mg, not more than 6 mg, not more than 5 mg, not more than 4 mg.

The dosage of rapamycin or a rapamycin derivative per adult per day is, for example, preferably 0.1 mg to 10 mg, preferably 0.1 mg to 9 mg, preferably 0.2 mg to 8 mg, preferably 0.4 mg to 7 mg, preferably 0.8 mg to 6 mg, more preferably 1 mg to 5 mg, more preferably 2 mg to 4 mg.

With rapamycin or a rapamycin derivative in any of the above amounts, Fabry disease can be effectively treated.

The dosage form of the internal medicine is not limited, and can be, for example, tablet, capsule, powder, dispersible tablet, syrup, or the like.

### [(II) External analgesic]

Pains to be treated by an external analgesic in accordance with one embodiment of the present invention is not particularly limited, and examples include nervous pains, Fabry disease-derived pains, back pain, inflammatory pains, cancerous pains, and pains derived from other causes. The term "external analgesic" herein refers to an analgesic in the form of external medicine.

Examples of diseases with pain, for which the external analgesic in accordance with one embodiment of the present invention is effective, include neurofibroma, shingles, trigeminal neuralgia, and back pain. The external analgesic in accordance with one embodiment of the present invention also works on pains that cannot be relieved with publicly known external analgesics, and puts fewer burdens on living bodies.

For example, in a case where neurofibromas grew with major nerves such as sciatic nerve involved therein, surgically removing the neurofibromas would place a large burden on the living body and is not realistic. In other words, the pain of neurofibromas may be untreatable with any surgical means. On the other hand, external medicines would place fewer burdens on the living body.

The external analgesic in accordance with one embodiment of the present invention can be achieved, for example, in the same manner as described in regard to the foregoing agent for treating Fabry disease in the form of external medicine. That is, the external analgesic in accordance with one embodiment of the present invention can be achieved in the same manner as described in the foregoing section "(I) Agent for treating Fabry disease" (more specifically, in the sections "(I-A) External medicine", "(I-A-1) Gel preparation", and "(I-A-2) Ointment").

### [(III) Perspiration enhancer]

Subjects to be treated by a perspiration enhancer in accordance with one embodiment of the present invention are, for example, patients with diminished sweating and patients with absent sweating. Examples include patients with anhidrosis or hypohidrosis. Other examples include Fabry disease. Also included are local anhidrosis and local hypohidrosis, in which absent sweating or diminished sweating is occurring in only a limited part. In a case of anhidrosis or hypohidrosis in which absent sweating or diminished sweating is occurring in a wide area of the skin, the patient is unable to regulate body temperature, and the patient's body temperature is likely to rise in summer and decrease in winter. Infants often suffer from fever cramps and epilepsy, and may develop acute encephalopathy.

The inventors previously found that rapamycin has an antiperspirant effect. However, surprisingly, it was found that, when used in conditions with diminished sweating, rapamycin has a perspiration enhancing effect, instead of the perspiration reducing effect.

The perspiration enhancer in accordance with one embodiment of the present invention can be achieved, for example, in the same manner as described in regard to the foregoing agent for treating Fabry disease. That is, the perspiration enhancer in accordance with one embodiment of the present invention can be achieved in the same manner as described in the foregoing sections "(I) Agent for treating Fabry disease", "(I-A) External medicine", "(I-A-1) Gel preparation", "(I-A-2) Ointment", and "(I-B) Internal medicine".

### [Presumed action mechanism]

The following description will discuss, with reference to Fig, 10, a presumed action mechanism of how rapamycin or a rapamycin derivative brings about the foregoing effects. It should be noted that the presumed action mechanism described herein is an example to help understanding of the present invention, and is not intended to limit the scope of the present invention in any way.

In a nerve cell of central nervous system in normal condition, mTORC1 directly activates AMPAR to accelerate uptake of calcium, and in turn accelerates the activation of NMDAR that occurs subsequently to the activation of AMPAR, resulting in the acceleration of the influx of calcium ions into the nerve cell. On the other hand, calcium/calmodulin-dependent protein kinase II (CaMKII) also accelerates the influx of calcium ions into the nerve cell by increasing the mean open time of NMDAR. Meanwhile, mTORC1 inhibits phosphorylation of CaMKII and thereby regulates the activation of the nerve cell ((a) of Fig. 10).

In a nerve cell of central nervous system of a Fabry disease patient, accumulated globotriaosylceramide (Gb3) or accumulated globotriaosylsphingosine (Lyso-Gb3), which is a kind of glycolipid, increases the mean open time of NMDAR of the nerve cell, and thereby accelerates the influx of calcium ions into the nerve cell. Therefore, in the Fabry disease patient, the influx of calcium ions into nerve cells is excessive ((b) of Fig. 10).

As described above, in the Fabry disease patient, the influx of calcium ions into nerve cells is excessive. The administration of rapamycin inhibits the action of mTORC1 and, in turn, reduces the influx of calcium ions and, at the same time, accelerates the phosphorylation of CaMKII and thus accelerates a reduction in calcium ions excessively accumulated within the cytoplasm. Furthermore, a reduction in amount of unphosphorylated CaMKII, which results from the acceleration of the phosphorylation of CaMKII, reduces the influx of calcium ions through NMDAR, and encourages the reduction of calcium ions within the cytoplasm ((c) of Fig. 10). It is inferred from this that the administration of rapamycin to a Fabry disease patient is effective. Furthermore, as is clear from Fig. 10, similar effects are expected from other mTORC1 inhibitors.

### Examples

### <Production Examples: Preparation of rapamycin-containing gel preparation>

Gel preparations containing rapamycin at various concentrations were prepared. Specifically, sirolimus (rapamycin) was added and dissolved in ethanol, and then water (specifically, water for injection) was further added and mixed to obtain a mixed solution. To this mixed solution, Carbopol (registered trademark) (specifically, Carbopol (registered trademark) 934P NF) was added and mixed to obtain a homogeneous suspension. To the suspension, a neutralizer (specifically, tris(hydroxymethyl)aminomethane) was added and mixed to obtain a gel preparation.

Components other than rapamycin, contained in 1 g of the above gel, were as follows: 16 mg of Carbopol (registered trademark) 934P NF; 490 mg of water; 480 mg to 490 mg of ethanol; and 6 mg of tris(hydroxymethyl)aminomethane.

More specifically, gel preparations were prepared with the compositions of the following Production Examples 1 and 2 and Comparative Production Example 1.

### Production Example 1: Gel containing 0.4 wt% rapamycin (total quantity: 100g)

Rapamycin: 0.4 g
Ethanol: 48.4 g
Water for injection: 49 g
Carbopol (registered trademark) 934P NF: 1.6 g
Tris(hydroxymethyl)aminomethane: 0.6 g

### Production Example 2: Gel containing 0.8 wt% rapamycin (total quantity: 100 g)

Rapamycin: 0.8 g
Ethanol: 48 g
Water for injection: 49g
Carbopol (registered trademark) 934P NF: 1.6g
Tris(hydroxymethyl)aminomethane: 0.6 g

### Comparative Production Example 1: Base gel containing no rapamycin

Ethanol: 48.4 g
Water for injection: 49 g
Carbopol (registered trademark) 934P NF: 1.6 g
Tris(hydroxymethyl)aminomethane: 0.6 g

In the following descriptions, the expression "X% rapamycin gel" in Examples refers to "gel containing X wt% rapamycin" (X represents a number), unless otherwise specifically noted.

### [Example 1: Verification of effects of rapamycin-containing external medicine on wild-type mouse]

The following experiments were conducted on rapamycin-administered wild-type mice in regard to perception of pain stimulation and amount of sweating.

### [Test Method 1-1]

A 0.4% rapamycin gel or a base gel was applied to plantar regions of hind paws of three wild-type mice of each group.

Next, each mouse was placed on a hot plate heated to 60°C, and the time that elapsed before the mouse avoided pain was measured.

A pressure was applied to three wild-type mice of each group, and the pressure at a point in time in which each mouse avoided pain was measured. Specifically, a pressure was applied to the plantar region of the hind paw of each mouse, the pressure was gradually increased, and the pressure at a point in time in which the mouse avoided pain was measured, with the use of Von Frey pain assessment device manufactured by UGO BASILE.

### <Test Result 1-1>

(a) of Fig. 1 shows graphs illustrating comparisons, in time that elapsed before mice avoided pain and pressure at the time of pain avoidance, between wild-type mice who received application of a 0.4% rapamycin gel (left) and wild-type mice who received application of a base gel (right). The graph (a-1) shows reactions to thermal stimulation, and the graph (a-2) shows reactions to pressure stimulation. With the application of a rapamycin-containing gel, the time that elapsed before wild-type mice avoided pain (thermal stimulation) was significantly prolonged. On the other hand, the pressure at a point in time in which the wild-type mice avoided pain (pressure stimulation) showed increasing tendency.

### [Test Method 1-2]

The amount of sweating of wild-type mice was measured and mTOR activity in sweat gland tissue was checked.

### [Measurement of amount of sweating by iodine-starch method]

A 0.4% rapamycin gel, a 0.8% rapamycin gel, or a base gel was applied to three wild-type mice of each group. After 60 minutes from the application, general anesthesia was applied to each mouse by intraabdominally injecting Domitor, Betorufaru, and Dormicum. Furthermore, acetylcholine serving as a sudorific was administered to each mouse.

Next, a known iodine-starch method was used to measure changes in amount of sweating. First, mineral oil having an iodine ethanol solution and starch dissolved therein was applied to a plantar region of a hind paw of each mouse. After that, pilocarpine hydrochloride (50 µg/20 µL per mouse) was hypodermically injected to the plantar region of the hind paw of the mouse. The plantar region of the hind paw of the mouse was checked five minutes after the injection, and the number of black dots appeared on the plantar region of the hind paw (more specifically, sweat glands existing in a paw pad in the plantar region) was counted. Note that the black dots are regions that had a positive reaction to the iodine-starch test.

### [Observation of active mTOR by antibody staining]

A 0.4% rapamycin gel or a base gel was applied to three wild-type mice of each group. The sweat gland tissue of each mouse was stained with an antibody to active mTOR, and the activation state of mTOR was checked.

The mice were generally anesthetized by intraabdominally injecting, per 1 kg of the body weight of each mouse, 0.3 mg of Domitor, 5 mg of Betorufaru, and 4 mg of Dormicum.

Next, acetylcholine (pilocarpine hydrochloride' Junsei Chemical, Co., Ltd. Tokyo, Japan) serving as a sudorific was dissolved in phosphate buffered saline, and thereby a stimulating solution (concentration of acetylcholine: 50 µg/µL) was prepared.

To a hind paw of the mouse under general anesthesia, 10 µL of the stimulating solution was hypodermically injected. Two minutes after the injection, a skin sample was taken from the hind paw of the mouse, and the skin sample was embedded in an OCT compound (Lab-Tek Products, Illinois, USA). From the OCT compound, a section having a thickness of 5 pm was prepared, and the section was stained in accordance with a known fluorescent staining method.

A primary antibody used in the fluorescent staining method was anti-phospho-mTOR (ser2448) (1 : 50, Cell Signaling Technology, Tokyo, Japan). Note that anti-phospho-mTOR is an antibody which recognizes phosphorylated serine at the 2448th position from the amino terminal of an mTOR protein. In other words, anti-phospho-mTOR is an antibody which recognizes an activated mTOR protein. A secondary antibody used in the fluorescent staining method was a commercially available secondary antibody (Thermo Fisher) liked with Alexa Fluor 488. Meanwhile, the above-mentioned section was stained with Hoechst 33342, and thereby cell nuclei in the section were stained. An image of fluorescent-stained portions was observed with use of a BZ-8000 microscope (Keyence).

### [Observation of active mTOR by western blotting]

A 0.4% rapamycin gel or a base gel was applied to plantar regions of hind paws of three wild-type mice of each group. The sweat gland tissue of each mouse was subjected to western blotting, and the amount of active mTOR contained in the sweat gland tissue was checked.

The mice were generally anesthetized by intraabdominally injecting, per 1 kg of the body weight of each mouse, 0.3 mg of Domitor, 5 mg of Betorufaru, and 4 mg of Dormicum.

Next, acetylcholine (pilocarpine hydrochloride' Junsei Chemical, Co., Ltd. Tokyo, Japan) serving as a sudorific was dissolved in phosphate buffered saline, and thereby a stimulating solution (concentration of acetylcholine: 50 µg/µL) was prepared.

To a hind paw of the mouse under general anesthesia, 10 pL of the stimulating solution was hypodermically injected. Two minutes after the injection, a skin sample was taken from the hind paw of the mouse, and the skin sample was subjected to western blotting.

A primary antibody used in western blotting was Rabbit anti-pmTOR antibody available from Cell Signaling Technology (CST), and a second antibody used in western blotting was HRP-binding Anti-rabbit IgG antibody.

### <Test Result 1-2>

(b) of Fig. 1 shows the test results of [Measurement of amount of sweating by iodine-starch method].

(b) of Fig. 1 shows the number of black dots on mice who received application of a base gel (left), a 0.4% rapamycin gel (middle), and a 0.8% rapamycin gel (right). The graph indicates that, with the application of a rapamycin-containing gel, the amount of sweating of each wild-type mouse significantly decreased to an extent that depends on the concentration of contained rapamycin.

(a) of Fig. 2 shows the test results of [Observation of active mTOR by antibody staining].

As is clear from (a) of Fig. 2, stained areas of the image of active mTOR and stained areas of the image of cell nuclei match each other (see Merge). This result confirms that the stained image of active mTOR is not a background (i.e., extracellular matrix or the like nonspecifically stained) but an image of specifically stained active mTOR within cells. Furthermore, as is clear from (a) of Fig. 2, in a case where a rapamycin gel was used, the staining intensity of active mTOR was weaker than a case where a base gel was used. That is, this reveals that, in a case where a rapamycin gel is used, the amount of active mTOR is small as compared to a case in which a base gel is used.

(b) of Fig. 2 shows the test results of [Observation of active mTOR by western blotting].

(b) of Fig. 2 also revealed that, in a case where a rapamycin gel is used, the amount of active mTOR (p-mTOR) is small as compared to a case in which a base gel is used.

### [Example 2: Confirmation of symptoms of Fabry disease model mouse]

The following experiments were conducted on Fabry disease model mice in regard to perception of pain stimulation and amount of sweating. The Fabry disease model mice used here were α-gal-knockout mice (B6;129-Gla tm1Kul/J) purchased from Jackson Laboratory.

### [Test Method 2-1]

Three wild-type mice and three Fabry disease model mice were prepared.

The subsequent processes of this experiment were carried out in the same manner as described in the [Test Method 1-1] section, i.e., in the same manner as the experiment of placing each mouse on a hot plate heated to 60°C and measuring the time that elapsed before the mouse avoided pain.

### <Test Result 2-1>

(a) of Fig. 3 is a graph illustrating a comparison in time that elapsed before mice avoided pain between wild-type mice (left) and Fabry disease model mice (right). The graph indicates that the time that elapsed before Fabry disease model mice avoided pain tends to be short.

### [Test Method 2-2]

The amount of sweating of wild-type mice and Fabry disease model mice was measured, and active mTOR in sweat gland tissue in each mouse was observed.

### [Measurement of amount of sweating by iodine-starch method, and observation of active mTOR by antibody staining]

Three wild-type mice and three Fabry disease mode mice were prepared, and amount of sweating was measured. The sweat gland tissue of each of the wild-type mice and Fabry disease model mice was stained with an antibody to active mTOR, and the activation state of mTOR was checked.

This experiment was carried out in the same manner as described in the foregoing [Measurement of amount of sweating by iodine-starch method] section and the foregoing [Observation of active mTOR by antibody staining] section.

### <Text Result 2-2>

(b) of Fig. 3 is a graph illustrating a comparison in the number of black spots between wild-type mice (left) and Fabry disease model mice (right). The graph indicates that the amount of sweating of Fabry disease model mice is significantly less than that of wild-type mice (p = 0.0122).

(c) of Fig. 3 shows images of fluorescent-stained sweat gland of a wild-type mouse and a Fabry disease model mouse. In strongly stained areas, a lot of active mTOR is expressed. The images revealed that more active mTOR is expressed in the sweat gland tissue of the Fabry disease model mouse than in the sweat gland tissue of the wild-type mouse.

### [Example 3: Verification of effects of rapamycin-containing external medicine on Fabry disease model mouse]

The following experiments were conducted on rapamycin-administered Fabry disease model mice in regard to perception of pain stimulation and amount of sweating.

### [Text Method 3-1]

A 0.4% rapamycin gel or a base gel was applied to plantar regions of hind paws of three Fabry disease model mice of each group.

The subsequent processes of this experiment were carried out in the same manner as described in the [Test Method 1-1] section, i.e., in the same manner as the experiment of placing each mouse on a hot plate heated to 60°C and measuring the time that elapsed before the mouse avoided pain.

### <Test Result 3-1>

(a) of Fig. 4 is a graph illustrating a comparison in time that elapsed before mice avoided pain between Fabry disease model mice who received application of a base gel (left) and Fabry disease model mice who received application of a 0.4% rapamycin gel (right). The graph indicates that the Fabry disease model mice who received application of a rapamycin-containing gel showed a tendency that the time that elapsed before mice avoided pain, which had been shortened due to Fabry disease, is prolonged.

### [Test Method 3-2]

The amount of sweating of Fabry disease model mice who received application of a base gel or a rapamycin gel was measured. The mTOR activity in sweat gland tissue of each mouse was observed.

### [Measurement of amount of sweating by iodine-starch method, and observation of active mTOR by antibody staining]

A base gel or a 0.4% rapamycin gel was applied to plantar regions of hind paws of three Fabry disease model mice of each group, and amount of sweating was measured. The sweat gland tissue of each mouse was stained with an antibody to active mTOR, and the activation state of mTOR was checked.

This experiment was carried out in the same manner as described in the foregoing [Measurement of amount of sweating by iodine-starch method] section and the foregoing [Observation of active mTOR by antibody staining] section.

### <Test Result 3-2>

(b) of Fig. 4 is a graph illustrating a comparison in the number of black spots between Fabry disease model mice who received application of a base gel (left) and Fabry disease model mice who received application of a 0.4% rapamycin gel (right). The graph indicates that the Fabry disease model mice who received application of a rapamycin gel, who had had a decreased amount of sweating due to Fabry disease, showed a tendency that their amount of sweating increases.

(c) of Fig. 4 shows images of fluorescent-stained sweat gland of a Fabry disease model mouse who received application of a base gel and a Fabry disease model mouse who received application of a 0.4% rapamycin gel. In strongly stained areas, a lot of active mTOR is expressed. As shown in (c) of Fig. 4, it was revealed that the application of a rapamycin gel inhibits the activation of mTOR in the sweat gland tissue of the Fabry disease model mouse.

### [Example 4: Confirmation of expression of mTOR in angiokeratoma of Fabry disease patient]

The activation state of mTOR in an angiokeratoma of a Fabry disease patient, and the effects of ERT (conventional treatment method to treat Fabry disease) on an angiokeratoma, were checked. An enzyme used in ERT of this test was agalsidase beta, and specific processes of the treatment method were based on usual dosages.

### [Test Method]

A piece of the skin of a Fabry disease patient including an angiokeratoma lesion was stained with an anti-p-mTOR antibody, and the activation state of mTOR was checked.

### <Test Result>

(a) of Fig. 5 is an image of the stained angiokeratoma of the Fabry disease patient. The image indicates that, in the angiokeratoma of the Fabry disease patient, the horny layer has been thickened to a slight degree and that a capillary in the dermal papillary layer has become dilated to a great degree due to repletion of red blood cells.

(b) of Fig. 5 shows images of fluorescently-stained p-mTOR and cell nuclei (Ho) in the angiokeratoma of the Fabry disease patient. The images (b-1) of Fig. 5 were taken before ERT, and the images (b-2) of Fig. 5 were taken after ERT. It is clear from (b-1) of Fig. 5 that mTOR is active in the angiokeratoma of the Fabry disease patient before receiving ERT. It is clear from (b-2) of Fig. 5 that mTOR is also active in the angiokeratoma of the Fabry disease patient even after receiving ERT.

(c) of Fig. 5 show a series of images showing the transition of the condition of angiokeratomas of a Fabry disease patient after receiving ERT. (c) of Fig. 5 revealed that ERT is not effective in treating angiokeratomas.

It should be noted that the Fabry disease patient in the present example had a normal blood GL3 level and a normal urinary protein level, and no cardiovascular or cerebrovascular abnormalities were found.

### [Example 5: Verification of analgesic effect of rapamycin-containing external medicine on wild-type mouse]

The following experiment was conducted on rapamycin-administered wild-type mice in regard to perception of pain stimulation.

### [Test Method]

Three wild-type mice who had received application of a 0.8% rapamycin gel on their plantar regions of hind paws were prepared, three wild-type mice who had received application of a base gel on their plantar regions of hind paws were prepared, and three wild-type mice who had not received any application were prepared.

The subsequent processes of this experiment were carried out in the same manner as described in the [Test Method 1-1] section, i.e., in the same manner as the experiment of placing each mouse on a hot plate heated to 60°C and measuring the time that elapsed before the mouse avoided pain.

### <Test Result>

(a) of Fig. 6 is a graph showing a comparison in time that elapsed before mice avoided pain among wild-type mice who received application of a 0.8% rapamycin gel (left), wild-type mice who received application of a base gel (middle), and wild-type mice who received no application (right). The results revealed that the time that elapsed before mice avoided pain was prolonged by application of a rapamycin gel.

### [Example 6: Verification of analgesic effect of rapamycin-containing external medicine on healthy human subject]

The following experiment was conducted on a rapamycin-administered healthy human subject in regard to perception of pain stimulation.

### [Test Method]

A 0.4% rapamycin gel was applied to a part (part of a palm adjacent to the wrist) of the skin of the subject, and then electric current was passed through the area that had received the application of the rapamycin gel. The smallest value of the electric current that the subject could feel was measured. Specifically, the test was carried out with the use of a perception/pain quantitative analytical device PainVision PS-2100 (Nipro) in accordance with the protocol that came with the device.

### <Test Result>

(b) to (d) of Fig. 6 show the results of measurements of the smallest value of electric current at which the subject could feel pain. These graphs indicate that the rapamycin-containing external medicine reduces the threshold for pain stimulation also in humans.

### [Example 6: Effects of administration of rapamycin on peripheral nerves]

The distribution of nerve cells of a dorsal root ganglion in the skin (paw pad) of a wild-type mouse and a Fabry disease model mouse was observed by immunostaining. The effects of administration of rapamycin on nerve cells of a dorsal root ganglion of a Fabry disease model mouse were also checked.

### [Test Method 6-1]

A sample was cut from a paw pad of each of the wild-type and Fabry disease model mice, and fixed in 10% formaldehyde for 24 hours. Next, the sample was embedded in paraffin, and a section was prepared.

The section was immersed in Protein Block, Serum Free (manufactured by Dako) for 30 minutes to block non-specific antigens contained in the section. Next, the section was incubated overnight at 4°C with a primary antibody. Then, the section was incubated at room temperature for 60 minutes with a secondary antibody. The combination of primary and secondary antibodies used here was (1) a combination of rabbit anti-pan-neuronal marker PGP9.5 (manufactured by Sigma) and Alexa Fluor 554 Goat Anti-ribbit IgG (H+L) (manufactured by Life technology) or (2) a combination of anti-Beta III Tubulin in antibody diluent (manufactured by Dako) and FITC rabbit anti-chicken IgG (manufactured by Life technology). The combination (1) is used to stain entire nerve cells, and the combination (2) is used to stain axons.

Lastly, the cell nuclei in the section were stained with Hoechst 33342 (500 times diluted, manufactured by Invitrogen). The section after the staining was observed under a microscope (BZ-X710, manufactured by KEYENCE), and a stained image was taken.

### <Test Result 6-1>

(a) of Fig. 7 is an image of immunostained skin tissue of a paw pad of a wild-type mouse, and (b) of Fig. 7 is an image of immunostained skin tissue of a paw pad of a Fabry disease model mouse. A comparison in form of the nerve cells of dorsal root ganglia between these images indicates that the axons of the nerve cells are short in brain tissue of the Fabry disease mouse.

### [Test Method 6-2]

Wild-type mice were divided into three groups, and Fabry disease model mice were divided into three groups. A base alone was applied to one group, a 2% rapamycin-containing gel was applied to one group, and a 1% Voltaren-containing gel was applied to one group, and blood was collected after 24 hours from the application. The BDNF concentration in the blood was measured.

### <Test Result 6-2>

The BDNF concentration in blood of Fabry disease model mice remarkably increased ((c) of Fig. 7).

### [Test Method 6-3]

Dorsal root ganglion cells (DRG cells) were taken from a 20-week-old wild-type mouse. The dorsal root ganglion nerve cells thus taken were first subjected to extraction with Ham's 12 containing 30% Percoll, and then were cultured at 37°C for 2 days with the use of a Gibco Neurobasal-A medium (manufactured by Gibco) supplemented with 2 mM GlutaMax1, 2% B-27 w/o Vitamin A, 5 ng/mL recombinant BDNF carrier free, and 10 ng/mL recombinant human-beta NGF carrier free. Then, the culture medium was replaced with a medium supplemented with 2 mM GlutaMax1, 2% B-27 w/o Vitamin A, 10 pM Retinoic Acidn, and 100 ng/mL recombinant human-beta NGF carrier free, and then a comparative observation of the extension of axons was carried out with a microscope (BZ-X710, manufactured by KEYENCE), and microscope images were taken.

### <Test Result 6-3>

(d) of Fig. 7 is a microscope image taken after the administration of BDNF. As shown in the microscope image, the axons were found to have extended by the administration of BDNF in the DRG cells of the wild-type mouse. This suggests that the administration of rapamycin is effective in treating Fabry disease via BDNF.

### [Example 7: Inhibition of influx of calcium ions into cells by administration of rapamycin]

The effects of inhibiting the influx of calcium ions into cells by the administration of rapamycin were verified with the use of wild-type human neuroblastoma cells (SK-N-MC) and α-galA-knockout SK-N-MC. It should be noted that α-galA-knockout SK-N-MC can be regarded as Fabry disease model cells.

### [Test method]

The calcium level in cells was measured with the use of Calcium Kit-Fura2 (manufactured by Dojindo).

First, α-galA-knockout SK-N-MC was transferred to a 35 mm dish. After 24 hours from the transfer, the α-galA-knockout SK-N-MC was incubated at 37°C for 1 hour in a loading buffer containing Fura2-AM (specific composition: 0.04% Pluronic F-127, 1.25 mM Probenecid, and 5 mg/L Fura2-AM), thereby allowing the α-galA-knockout SK-N-MC to take up a recording medium.

The cells were washed twice with phosphate buffered saline (PBS), and then recording buffer (which came with Calcium Kit-Fura2, manufactured by Dojindo) containing 1.25 mM Probenecid was poured into the dish. Then, the dish was placed on a stage of a microscope (DIAPHOT 300, manufactured by Nikon).

A recording buffer containing 1 mM, 5 mM, or 10 mM of glutamate was contacted with the cells, and the fluorescence intensity at 510 nm was measured with the use of AQUA COSMOS/RATIO (Hamamatsu Photonics). The excitation wavelengths here were 340 nm and 380 nm, and the value of (fluorescence intensity at excitation wavelength of 340 nm/fluorescence intensity at excitation wavelength of 380 nm) was used as the calcium concentration of the cells.

### <Test Result>

(a) of Fig. 8 shows images showing the influx of calcium ions into cells. The results shown in (a) of Fig. 8 are: wild-type cells (upper left); rapamycin-administered wild-type cells (upper right); α-galA-knockout cells (lower left); and rapamycin-administered α-galA-knockout cells (lower right). Each of these results is for the case of contacting with 1 mM of glutamate. These images indicate that the amount of calcium ions moving into cells is larger in α-galA-knockout cells than wild-type cells. The images also indicate that the administration of rapamycin causes an increase in amount of calcium ions moving into cells in the case of wild-type cells but causes a decrease in amount of calcium ions into cells moving into cells in the case of α-galA-knockout cells.

(b) and (c) of Fig. 8 are graphs showing changes over time in influx of calcium ions into cells. (b) of Fig. 8 shows the result for the case where no rapamycin was administered, and (c) of Fig. 8 shows the results for the case where rapamycin was administered. The graphs indicate that the administration of rapamycin to α-galA-knockout cells causes a dramatic reduction in influx of calcium ions into cells. It should be noted that the controls shown in both graphs are SK-N-MC without treatment and SK-N-MC (control) knocked down with the use of a control plasmid.

### [Example 8: Verification of effects of rapamycin administration by MRI]

In the brains of wild-type and Fabry disease model mice, the activity of the region that perceives heat and pain was checked by manganese-enhanced MRI.

### [Test Method]

First, manganese chloride (MnCl₂) was intraabdominally administered to a wild-type mouse and a Fabry disease model mouse. Specifically, a 50 mM physiological saline (aqueous 0.9% sodium chloride solution) containing manganese chloride was intraabdominally administered three consecutive days so that the dosage was 30 mg/kg.

On the fourth day, MRI scanning was carried out with the use of a 11.7T vertical-bore Bruker Avance II imaging system (manufactured by Bruker Biospin) and a custom-made 15-mm diameter transmit/receive volume radio frequency (RF) coil (manufactured by m2m imaging). In doing so, the mice were anesthetized with isoflurane (manufactured by Abbott Laboratories) and respiratory signals were monitored using a physiological monitoring system (manufactured by SA Instruments, Inc.) The body temperature was maintained by passing warm water through a warming pad.

During the four-day experiment, the administration of manganese chloride, the administration of rapamycin, thermal stimulation, and imaging by MRI were carried out in accordance with the following plan. The manganese ions in the body travel with calcium ions, and thereafter remain in the body for about two weeks. Therefore, it is possible to know the influx of calcium ions by checking the manganese ion distribution by MRI.

**[Table 1]**

| | Day 1 | Day 2 | Day 3 | Day 4 |
|---|---|---|---|---|
| MnCl₂ | Yes | Yes | Yes | |
| Thermal stimulation | | Yes | Yes | Yes |
| Rapamycin | Yes | Yes | Yes | |
| MRI | | | | Yes |

The obtained data was analyzed through OsiriX Lite.

### <Test Result>

Fig. 9 shows the results of MRI. Fig. 9 indicates that, in a Fabry disease model mouse without treatment, the activity of the region that perceives heat and pain was high. However, in a Fabry disease model mouse who received percutaneous administration of rapamycin before thermal stimulation, the activity of that region was low and equivalent to the wild-type mouse without treatment.

### Industrial Applicability

The present invention can be used in, for example, treating Fabry disease.

## Claims

1. An agent for treating Fabry disease, the agent comprising, as an active ingredient, at least one selected from the group consisting of rapamycin and rapamycin derivatives.

2. The agent according to claim 1, wherein the agent comprises, as an active ingredient, at least one selected from the group consisting of rapamycin, everolimus, temsirolimus, ridaforolimus, and zotarolimus.

3. The agent according to claim 1 or 2, wherein the agent is an external medicine.

4. The agent according to any one of claims 1 to 3, wherein the agent is for use in treating a skin lesion of Fabry disease.

5. An external analgesic comprising, as an active ingredient, at least one selected from the group consisting of rapamycin and rapamycin derivatives.

6. The external analgesic according to claim 5, wherein the external analgesic comprises, as an active ingredient, at least one selected from the group consisting of rapamycin, everolimus, temsirolimus, ridaforolimus, and zotarolimus.

7. A perspiration enhancer comprising, as an active ingredient, at least one selected from the group consisting of rapamycin and rapamycin derivatives.

8. The perspiration enhancer according to claim 7, wherein the perspiration enhancer comprises, as an active ingredient, at least one selected from the group consisting of rapamycin, everolimus, temsirolimus, ridaforolimus, and zotarolimus.

9. The perspiration enhancer according to claim 7 or 8, wherein the perspiration enhancer is for treatment of anhidrosis or hypohidrosis.
